# EUROPEAN PATENT SPECIFICATION

(11) **EP 0 429 242 B1**
(45) Date of publication and mention of the grant of the patent: **14.06.1995**
(21) Application number: 90312407.1
(22) Date of filing: 14.11.1990
(51) Int. Cl.: C12P 21/08, C12N 15/13, C12N 5/10, A61K 39/395, G01N 33/574

(54) **Specific binding agents**
Spezifische bindende Wirkstoffe
Agents attachant spécifiques

(30) Priority: 17.11.1989 GB 8926045; 07.09.1990 GB 9019552
(43) Date of publication of application: 29.05.1991
(73) Proprietor: UNILEVER PLC, London EC4P 4BQ (GB); UNILEVER N.V., 3013 AL Rotterdam (NL)
(72) Inventor: Verhoeyen, Martine Elisa, Sharnbrook, Bedford, MK44 1LQ (GB)
(74) Representative: Butler, David John

(56) References cited:
- EP-A- 0 239 400
- EUROPEAN JOURNAL OF BIOCHEMISTRY, vol. 176, no. 2, 1988, pages 287-295; P. DE WAELE et al.: "Expression in non-lymphoid cells of mouse recombinant immunoglobulin directed against the tumour marker human placental alkaline phosphatase"
- IDEM
- THE LANCET, August 17, 1986, 350-353;
- THE BRITISCH JOURNAL OF CANCER, 64 (1991), 911-914

## Description

This invention relates to specific binding agents, and in particular to polypeptides containing amino acid sequences that bind specifically to other proteinaceous or non-proteinaceous materials. The invention most particularly concerns the production of such specific binding agents by genetic engineering.

### Antibody structure

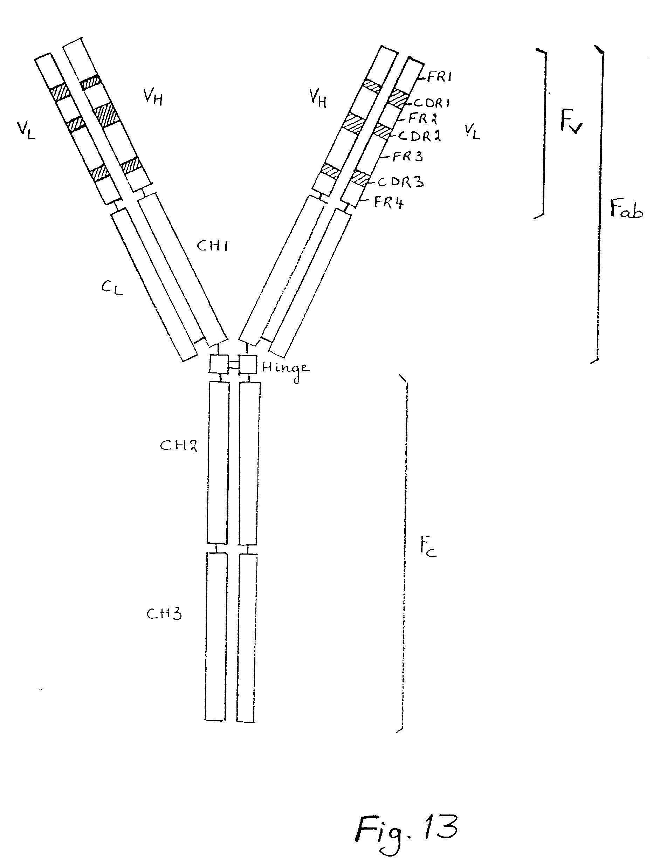

Natural antibody molecules consist of two identical heavy-chain and two identical light-chain polypeptides, which are covalently linked by disulphide bonds. Figure 13 of the accompanying drawings diagramatically represents the typical structure of an antibody of the IgG class. Each of the chains is folded into several discrete domains. The N-terminal domains of all the chains are variable in sequence and therefore called the variable regions (V-regions). The V-regions of one heavy (VH) and one light chain (VL) associate to form the antigen-binding site. The module formed by the combined VH and VL domains is referred to as the Fv (variable fragment) of the antibody. The C-terminal ends of both heavy and light chains are more conserved in sequence and chains are more conserved in sequence and therefore referred to as the constant regions. Heavy chain constant regions are composed of several domains, eg. the heavy chain of the gamma-isotype (IgG) consists of three domains (CH1, CH2, CH3) and a hinge region which connects the CH1 and CH2 domains. The hinges of the two heavy chains are covalently linked together by disulphide bridges. Light chains have one constant domain which packs against the CH1 domain. The constant regions of the antibody molecule are involved in effector functions such as complement lysis and clearing by Antibody Dependant Cell Cytotoxicity (ADCC). Classical digestion of an antibody with the protease papain yields three fragments. One fragment contains the CH2 and CH3 domains and, as it crystallises easily, was called the Fc fragment. The other two fragments were designated the Fab (antigen-binding) fragments, they are identical and contain the entire light chain combined with the VH and CH1 domain. When using pepsin, the proteolytic cleavage is such that the two Fabs remain connected via the hinge and form the (Fab)₂ fragment. Each of the domains is represented by a separate exon at the genetic level.

The variable regions themselves each contain 3 clusters of hypervariable residues, in a framework of more conserved sequences. These hypervariable regions interact with the antigen, and are called the Complementarity Determining Regions (CDRs). The more conserved sequences are called the Framework Regions (FRs). See Kabat et al (1987). X-ray studies of antibodies have shown that the CDRs form loops which protrude from the top of the molecule, whilst the FRs provide a structural beta-sheet framework.

### Modified antibodies

In one embodiment, the invention relates to so-called "reshaped" or "altered" human antibodies, ie. immunoglobulins having essentially human constant and framework regions but in which the complementarity determining regions (CDRs) correspond to those found in a non-human immunoglobulin, and also to corresponding reshaped antibody fragments.

The general principles by which such reshaped human antibodies and fragments may be produced are now well-known, and reference can be made to Jones et al (1986), Riechmann et al (1988), Verhoeyen et al (1988), and EP-A-239400 (Winter). A comprehensive list of relevant literature references is provided later in this specification.

Reshaped human antibodies and fragments have particular utility in the in-vivo diagnosis and treatment of human ailments because the essentially human proteins are less likely to induce undesirable adverse reactions when they are administered to a human patient, and the desired specificity conferred by the CDRs can be raised in a host animal, such as a mouse, from which antibodies of selected specificity can be obtained more readily. The variable region genes can be cloned from the non-human antibody, and the CDRs grafted into a human variable-region framework by genetic engineering techniques to provide the reshaped human antibody or fragment. To achieve this desirable result, it is necessary to identify and sequence at least the CDRs in the selected non-human antibody, and preferably the whole non-human variable region sequence, to allow identification of potentially important CDR-framework interactions.

### Summary of the invention

Monoclonal antibodies having specificity for human placental alkaline phosphatase (PLAP), an important cancer marker, are known per se. DeWaele et al., Eur. J. Biochem. 176, 287-295 describe the expression of functional immunoglobulins by COS or CHO cells transfected with plasmids encoding the heavy and light chain of anti-PLAP murine monoclonal antibody E6, and diagnostic and therapeutic applications of the antibody. Epenetos et al., The Lancet, August 17 1985, 350-353 describe cancer imaging experiments using radio-labelled anti-PLAP murine monoclonal antibody H17E2.

The invention provides as an important embodiment, a reshaped human antibody, or a reshaped human antibody fragment, having specificity for human placental alkaline phosphatase (PLAP) conferred by the presence of one or more of the amino acid sequences:
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr

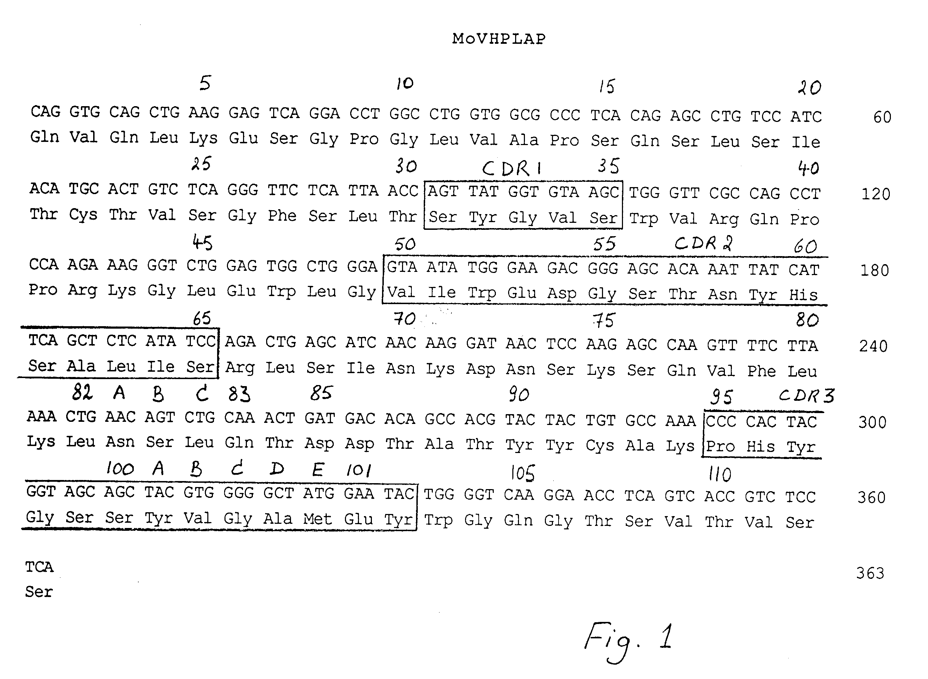

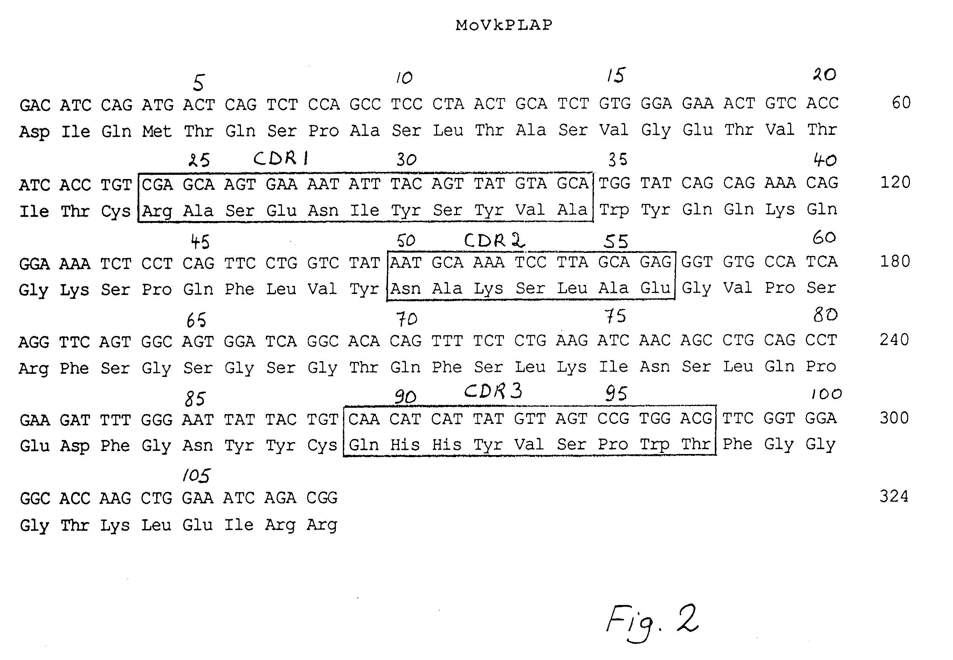

These amino acid sequences correspond to the CDRs depicted in Figures 1 and 2 of the accompanying drawings. Preferably, the reshaped antibody or fragment of the invention contains all 3 of the CDRs depicted in Figure 1 of the accompanying drawings, in a human heavy chain variable region framework. Alternatively, or in addition, the reshaped antibody or fragment of the invention contains all 3 of the CDRs depicted in Figure 2 of the accompanying drawings, in a human light chain variable region framework.

An antibody/fragment in accordance with the invention is therefore a synthetic specific binding polypeptide having specificity for human placental alkaline phosphatase (PLAP). This can be produced by recombinant DNA technology. Alternatively, the synthetic polypeptide can be produced by artificially assembling a sequence of amino acids. The synthetic polypeptide can be equivalent to an intact conventional antibody, or equivalent to a multiple or single-chain fragment of such an antibody, or can be simply a material that includes one or more sequences that confer the desired specific binding capability.

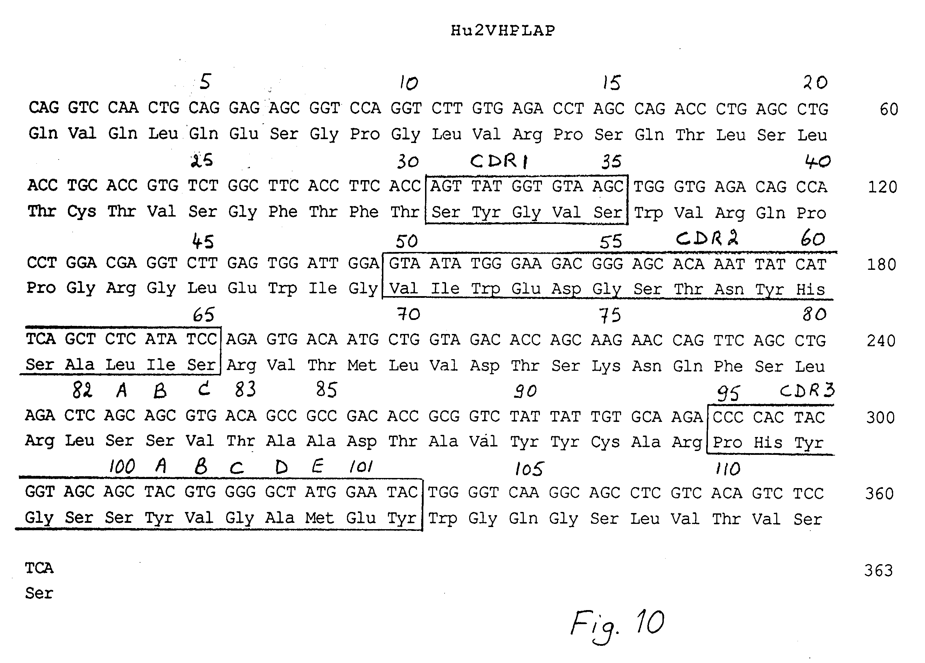

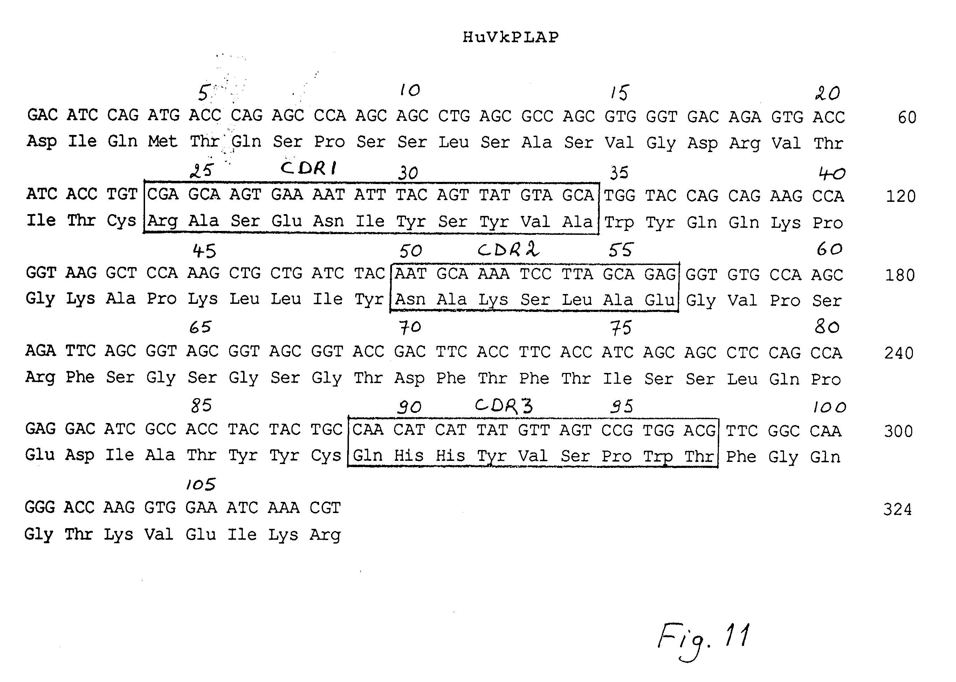

Another embodiment of the invention is a reshaped antibody or reshaped antibody fragment containing a protein sequence as depicted in Figure 10 and/or Figure 11 of the accompanying drawings.

Other important embodiments of the invention are an expression vector incorporating a DNA sequence as depicted in Figure 10 and/or Figure 11 of the accompanying drawings, and an expression vector incorporating a DNA sequence encoding one or more of the protein sequences designated as being a CDR in Figure 1 and/or Figure 2 of the accompanying drawings.

An important aspect of the invention is a stable host cell line containing a foreign gene that causes the host cell line to produce a specific binding agent according to the invention. This can be a stable host cell line containing a foreign gene that encodes at least one of the amino acid sequences designated as being a CDR in Figure 1 and/or Figure 2 of the accompanying drawings, together with a protein framework that enables the encoded amino acid sequence when expressed to function as a CDR having specificity for PLAP.

The invention particularly provides an immortalised mammalian cell line, or a yeast, or other eukaryotic cell, or a prokaryotic cell such as a bacterium, producing a reshaped antibody or fragment according to the invention.

Another important aspect of the invention is a synthetic specific binding agent, reshaped human antibody or reshaped human antibody fragment, having specificity equivalent to that of the gamma-1, kappa anti-PLAP monoclonal antibody secreted by murine hybridoma cell line H17E2.

The invention also provides two novel plasmids, pSVgptHu2VHPLAP-HuIgG1 and pSVneoHuVkPLAP-HuCk, and these plasmids can be used in the production of a synthetic specific binding agent, reshaped human antibody or reshaped human antibody fragment.

These plasmids are contained in novel E.coli strains NCTC 12389 and NCTC 12390, respectively.

Other aspects of the invention are:
a) A DNA sequence encoding a reshaped human antibody heavy-chain variable region having specificity for human placental alkaline phosphatase, as contained in E.coli NCTC 12389.
b) A DNA sequence encoding a reshaped human antibody light-chain variable region having specificity for human placental alkaline phosphatase, as contained in E.coli NCTC 12390.
c) A reshaped human antibody heavy-chain variable region having specificity for human placental alkaline phosphatase, producible by means of the expression vector contained in E.coli NCTC 12389.
d) A reshaped human antibody light-chain variable region having specificity for human placental alkaline phosphatase, producible by means of the expression vector contained in E.coli NCTC 12390.
e) A reshaped human antibody or reshaped human antibody fragment, comprising at least one variable region according to c) or d) above.

A particular embodiment of the invention is therefore a reshaped human antibody or fragment possessing anti-PLAP specificity and incorporating a combination of CDRs (which may, for example, be cloned from a murine anti-PLAP immunoglobulin) having the amino acid sequences identified as CDR1, CDR2 and CDR3 respectively in Figures 1 and 2 of the accompanying drawings, which respectively represent the heavy chain variable region (VH) and light chain variable region (Vk) of a murine anti-PLAP monoclonal antibody that we have cloned and sequenced. In the case of an intact antibody, or a fragment comprising at least one heavy chain variable region and at least one light chain variable region, the reshaped antibody or fragment preferably contains all six CDRs from the non-human source. To be most effective in binding, the CDRs should preferably be sited relative to one another in the same arrangement as occurs in the original non-human antibody, e.g. the VH CDRs should be in a human VH framework, and in the order in which they occur naturally in the non-human antibody.

As will be apparent to those skilled in the art, the CDR sequences and the surrounding framework sequences can be subject to minor modifications and variations without the essential specific binding capability being significantly reduced. Such minor modifications and variations can be present either at the genetic level or in the amino acid sequence, or both. Accordingly, the invention encompasses synthetic (reshaped) antibodies and fragments that are functionally equivalent to those described herein having precisely defined genetic or amino acid sequences.

The invention can also be applied in the production of bi-specific antibodies, having two Fab portions of different specificity, wherein one of the specificities is conferred by a reshaped human variable chain region incorporating one or more of the CDRs depicted in Figures 1 and 2 of the accompanying drawings.

The invention can also be applied in the production of so-called single-chain antibodies (for example, as disclosed in Genex EP-A-281604), and also to polysaccharide-linked antibodies (see Hybritech EP-A-315456) and other modified antibodies.

Any human constant regions (for example, gamma 1, 2, 3 or 4-type) can be used.

Antibody fragments retaining useful specific binding properties can be (Fab)₂, Fab, Fv, VH or Vk fragments. These can be derived from an intact reshaped antibody, for example by protease digestion, or produced as such by genetic engineering.

### Practical applications of the invention

An important aspect of the invention is a reshaped human anti-PLAP antibody or fragment, as defined above linked to or incorporating an agent capable of retarding or terminating the growth of cancerous cells, or to an imaging agent capable of being detected while inside the human body. The invention also includes injectable compositions comprising either of such combinations in a pharmaceutically acceptable carrier, such as saline solution, plasma extender or liposomes. The invention also includes the use, in a method of human cancer therapy or imaging, of a reshaped human anti-PLAP antibody or fragment as defined above. The invention further includes the use of such an antibody or fragment for the manufacture of a medicament for therapeutic application in the relief of cancer in humans, or the use of such an antibody or fragment in the manufacture of a diagnostic composition for in-vivo diagnostic application in humans.

The Fc region of the antibody, itself using pathways and mechanisms available in the body, such as complement lysis and antibody dependent cellular cytotoxicity, can be used to affect adversely the growth of cancerous cells. In this embodiment, no additional reagent need be linked to the reshaped antibody.

Examples of agents capable of affecting adversely the growth of cancerous cells include radioisotopes, such as Yttrium 90 and Iodine 131; drugs such as methotrexate; toxins such as ricin or parts thereof; and enzymes which may for example turn an inactive drug into an active drug at the site of antibody binding.

Examples of imaging agents include radioisotopes generating gamma rays, such as Indium 111 and Technetium 99; radioisotopes generating positrons, such as Copper 64; and passive agents such as Barium which act as contrast agents for X-rays, and Gadolinium in nmr/esr scanning.

In order to link a metallic agent, such as a radioisotope, to a specific binding agent of the invention, it may be necessary to employ a coupling or chelating agent. Many suitable chelating agents have been developed, and reference can be made for example to US 4824986, US 4831175, US 4923985 and US 4622420. Techniques involving the use of chelating agents are described, for example, in US 4454106, US 4722892, Moi et al (1988), McCall et al (1990), Deshpande et al (1990) and Meares et al (1990).

The use of radiolabelled antibodies and fragments in cancer imaging and therapy in humans is described for example in EP 35265. It may be advantageous to use the radiolabelled cancer-specific antibody or fragment in conjunction with a non-specific agent radiolabelled with a different isotope, to provide a contrasting background for so-called subtraction imaging.

The antibody reagents of the invention can be used to identify, e.g. by serum testing or imaging, and/or to treat, PLAP-producing cancers. Such cancers can occur as, for example, breast cancer, ovarian cancer and colon cancer, or can manifest themselves as liquids such as pleural effusions.

### Modified antibody production

The portions of the VH and VL regions that by convention (Kabat, 1987) are designated as being the CDRs may not be the sole features that need to be transferred from the non-human monoclonal antibody. Sometimes, enhanced antibody performance, in terms of specificity and/or affinity, can be obtained in the reshaped human antibody if certain non-human framework sequences are conserved in the reshaped human antibody. The objective is to conserve the important three-dimensional protein structure associated with the CDRs, which is supported by contacts with framework residues.

The normal starting point from which a reshaped antibody in accordance with the invention can be prepared, is a cell (preferably an immortalised cell line), derived from a non-human host animal (for example, a mouse), which expresses an antibody having specificity against human PLAP. Such a cell line can, for example, be a hybridoma cell line prepared by conventional monoclonal antibody technology. Preferably, the expressed antibody has a high affinity and high specificity for PLAP, because it should be anticipated that some loss of affinity and/or specificity may occur during the transfer of these properties to a human antibody or fragment by the procedures of the invention. By selecting a high affinity and high specificity antibody as the parent antibody, the likelihood that the final reshaped antibody or fragment will also exhibit effective binding properties is enhanced.

The next stage is the cloning of the cDNA from the cell expressing the selected non-human antibody, and sequencing and identification of the variable region genes including the sequences encoding the CDRs. The procedures involved can now be regarded as routine in the art, although they are still laborious.

If the object is to produce a reshaped complete human antibody, or at least a fragment of such an antibody which will contain both heavy and light variable domains, it will be necessary to sequence the cDNA associated with both of these domains.

Once the relevant cDNA sequence or sequences have been analysed, it is necessary to prepare one or more replicable expression vectors containing a DNA sequence which encodes at least a variable domain of an antibody, which variable domain comprises human framework regions together with one or more CDRs derived from the selected non-human anti-PLAP antibody. The DNA sequence in each vector should include appropriate regulatory sequences necessary to ensure efficient transcription and translation of the gene, particularly a promoter and leader sequence operably linked to the variable domain sequence. In a typical procedure to produce a reshaped antibody or fragment in accordance with the invention, it may be necessary to produce two such expression vectors, one containing a DNA sequence for a reshaped human light chain and the other, a DNA sequence for a reshaped human heavy chain. The expression vectors should be capable of transforming a chosen cell line in which the production of the reshaped antibody or fragment will occur. Such a cell line may be, for example, a stable non-producing myeloma cell line, examples (such as NS0 and sp2-0) of which are readily available commercially. An alternative is to use a bacterial system, such as E.coli, as the expression vehicle for the reshaped antibody or fragment. The final stages of the procedure therefore involve transforming the chosen cell line or organism using the expression vector or vectors, and thereafter culturing the transformed cell line or organism to yield the reshaped human antibody or fragment.

By way of example only, detailed steps by means of which appropriate expression vectors can be prepared are given later in this specification. The manipulation of DNA material in a suitably equipped laboratory is now a well-developed art, and the procedures required are well within the skill of those versed in this art. Many appropriate genomic and cDNA libraries, plasmids, restriction enzymes, and the various reagents and media which are required in order to perform such manipulations, are available commercially from suppliers of laboratory materials. For example, genomic and cDNA libraries can be purchased from Clontech Laboratories Inc. The steps given by way of example below are purely for the guidance of the reader of this specification, and the invention is in no way critically dependant upon the availability of one or more special starting materials. In practice, the skilled person has a wide range of materials from which to choose, and can exploit and adapt the published technology using acquired experience and materials that are most readily available in the scientific environment. For example, many plasmids fall into this category, having been so widely used and circulated within the relevant scientific community that they can now be regarded as common-place materials.

### Examples

The procedure used to prepare reshaped anti-PLAP human antibodies is described in detail below, by way of example only, with reference to the accompanying drawings, of which:
Figure 1 shows the cDNA sequence coding for a murine heavy chain variable region having anti-PLAP specificity. The 3 classical CDRs are indicated, together with an amino acid sequence matching the cDNA code.
Figure 2 shows the cDNA sequence coding for a murine light chain variable region having anti-PLAP specificity.
Figures 3a and 3b together show a route by which an expression vector encoding a reshaped human heavy chain incorporating the CDRs of Figure 1, can be prepared.
Figures 4a and 4b together show a similar transformation route to obtain an expression vector encoding a reshaped human light chain incorporating the CDRs of Figure 2, can be prepared.
Figure 5 shows the plasmid pU12-IgEnh, which contains an enhancer sequence used in the routes of Figures 4a and 4b.
Figure 6 shows the source of plasmid pBGS18-HulgG1 used in the route of Figure 3b.
Figure 7 shows the source of plasmid pBGS18-HuCk used in the route of Figure 4b.
Figure 8 shows two synthetic oligonucleotide sequences I and II used in cloning the cDNA sequences of Figures 1 and 2.
Figure 9 shows six synthetic oligonucleotide sequences III to VIII used in the routes depicted in Figures 3a-4b.
Figures 10 and 11 show the cDNA and amino acid sequences of the resulting reshaped human heavy and light chain variable regions respectively.
Figure 12 shows in graphical form the relative specific anti-PLAP binding activity of the resulting reshaped human antibody.
Figure 13 depicts in diagrammatic form the structure of a typical antibody (immunoglobulin) molecule.

The experimental procedures required to practice the invention do not in themselves represent unusual technology, and they involve straightforward cloning and mutagenesis techniques as generally described for example in Verhoeyen et al (1988); Riechmann et al (1988) and EP-A-239400 (Winter). Alternatively, if an appropriate DNA sequence is already known in detail (the drawings accompanying this specification includes a sequence associated with anti-PLAP specificity), the reshaped human variable region genes can be synthesised in vitro (see Jones et al, 1986). Laboratory equipment and reagents for synthesising long oligonucleotides are readily available, and as techniques in this field develop it is becoming practicable to synthesise progressively longer sequences.

Detailed laboratory manuals, covering all basic aspects of recombinant DNA techniques, are available, e.g. "Molecular Cloning" by Sambrook et al (1989).

By means of the invention, the antigen binding regions of a mouse anti-PLAP antibody were grafted onto human framework regions. The resulting reshaped human antibody (designated Hu2PLAP) has binding characteristics similar to those of the original mouse antibody.

Such reshaped antibodies can be used for in vivo diagnosis and treatment of human cancers, eg. ovarian cancers and seminoma, and are expected at least to reduce the problem of an immune response in the patient often seen upon administration of non-human antibody. A similar benefit has been shown for reshaped CAMPATH-1 antibody in Hale et al (1988).

### Methods:

### a) Cloning and sequence determination of the mouse variable region genes

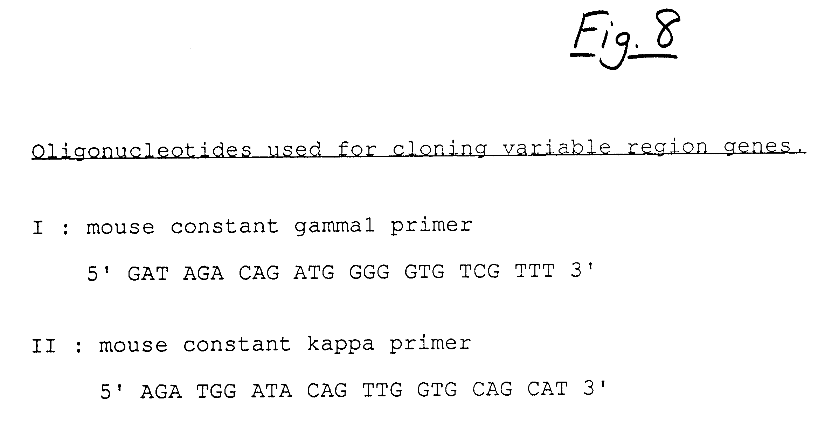

Messenger RNA was isolated from the murine hybridoma line "H17E2" which secretes a gamma-1, kappa anti-PLAP antibody, described in Travers et al (1984). First strand cDNA was synthesised by priming with oligonucleotides I and II (see Figure 8) complementary to the 5' ends of the CH1 and Ck exons respectively. Second strand cDNA was obtained as described by Gübler and Hoffmann (1983).

Kinased EcoRI linkers were ligated to the now double-stranded cDNA (which was first treated with EcoRI methylase, to protect possible internal EcoRI sites), followed by cloning into EcoRI-cut pUC9 (Vieira et al, 1982) and transformation of E.coli strain TG2 (Gibson, 1984).

Colonies containing genes coding for murine anti-PLAP VH (MoVHPLAP) and for murine anti-PLAP Vk (MoVkPLAP) were identified by colony hybridisation with 2 probes consisting respectively of 32P-labelled first strand cDNA of anti-PLAP VH and Vk. Positive clones were characterised by plasmid preparation, followed by EcoRI digestion and 1.5% agarose gel analysis. Full-size inserts (about 450bp) were subcloned in the EcoRI site of M13mp18 (Norrander et al, 1983). This yielded clones with inserts in both orientations, facilitating nucleotide sequence determination of the entire insert, by the dideoxy chain termination method (Sanger et al, 1977).

The nucleotide sequences, and their translation into amino acid sequences, of the mature variable region genes MoVHPLAP and MoVkPLAP, are shown in Figures 1 and 2. The 450 bp inserts included a signal sequence and 5' untranslated sequences and linkers, not shown in the Figures.

### b) Grafting of the mouse anti-PLAP CDRs onto human framework regions

The general techniques necessary to achieve this have been described very adequately in Jones et al (1986), Verhoeyen et al (1988), Riechmann et al (1988) and in EP-A-239400 (Winter).

The basic constructs used for reshaping were M13mp9HuVHLYS (Verhoeyen et al, 1988) and M13mp9HuVkLYS (Riechmann et al, 1988), which respectively contain the framework regions of the heavy chain variable region of human "NEW" and of the light chain variable region of human "REI". Both of these human antibodies have been thoroughly characterised and reported (Saul et al, 1978; and Epp et al, 1974, respectively).

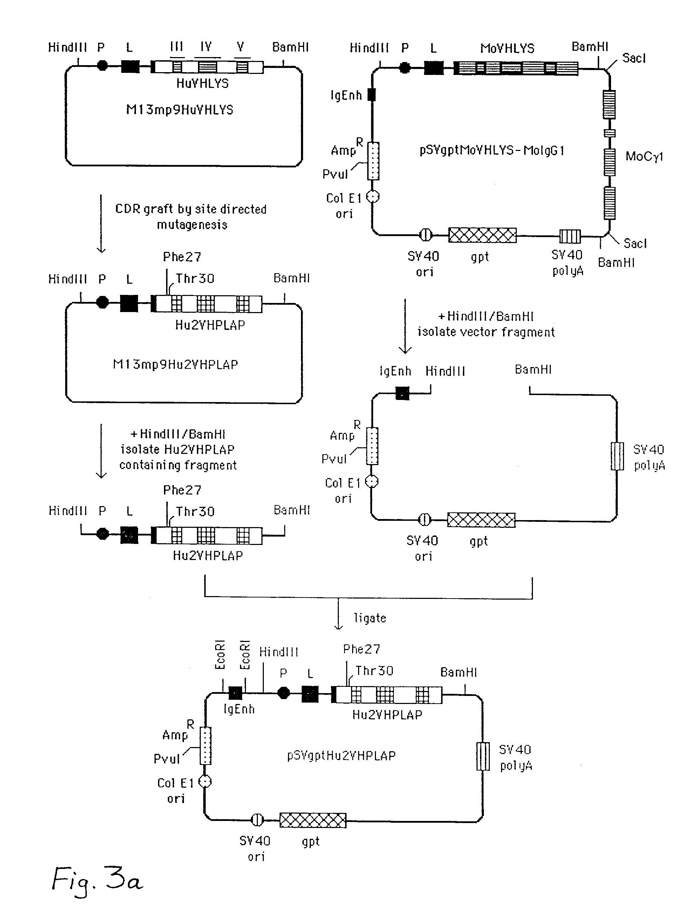

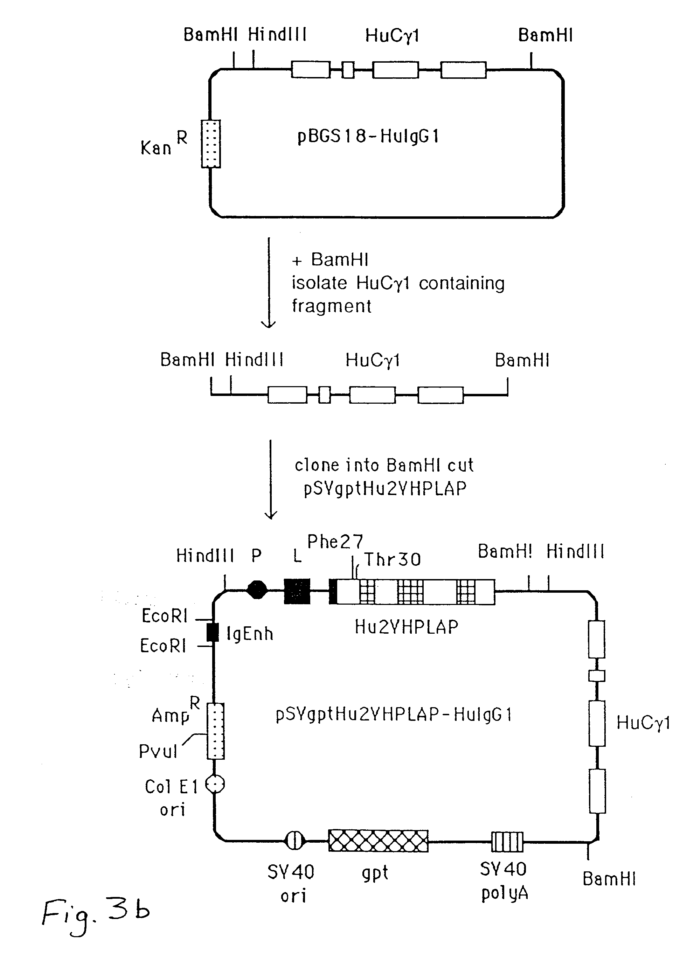

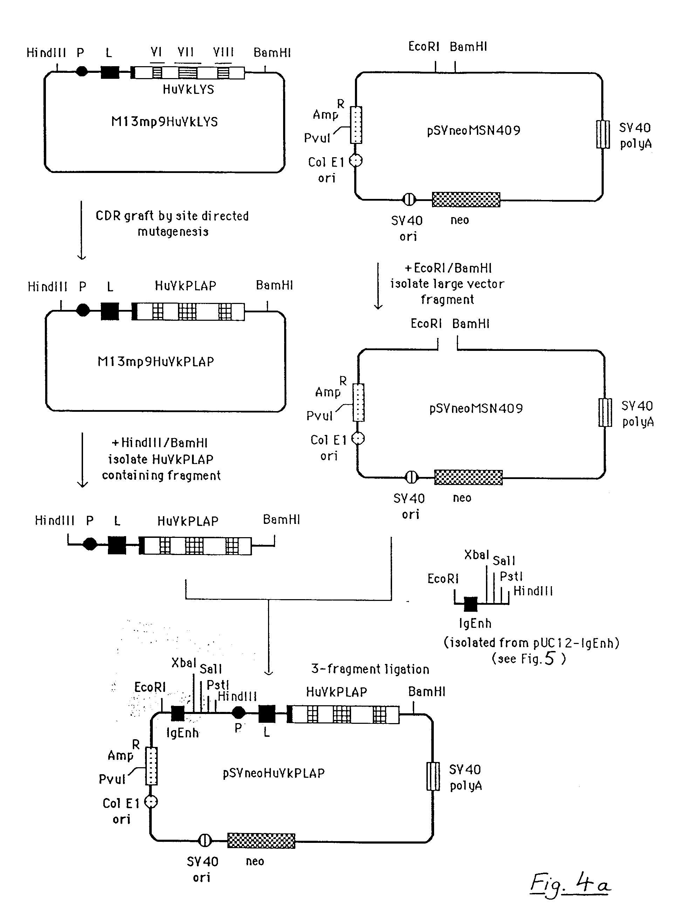

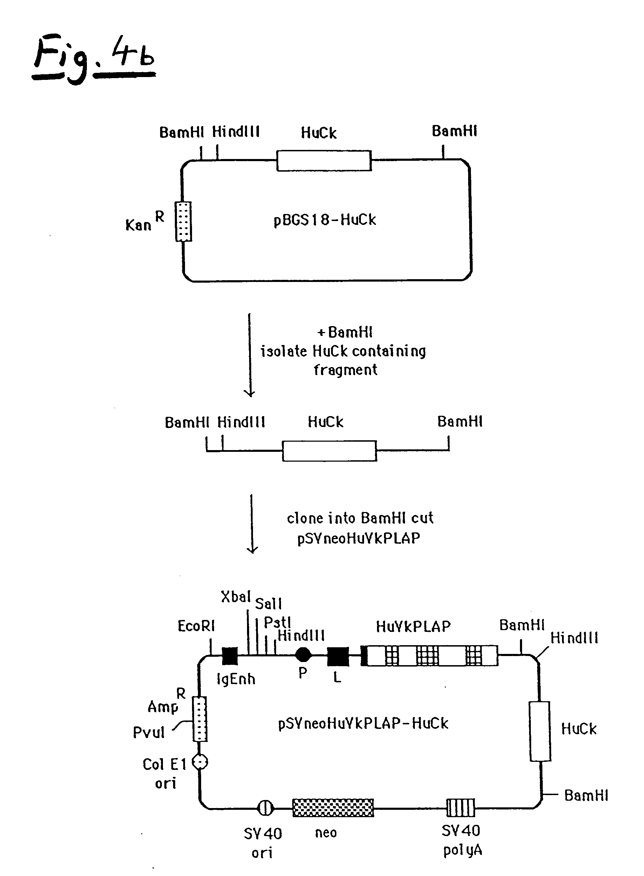

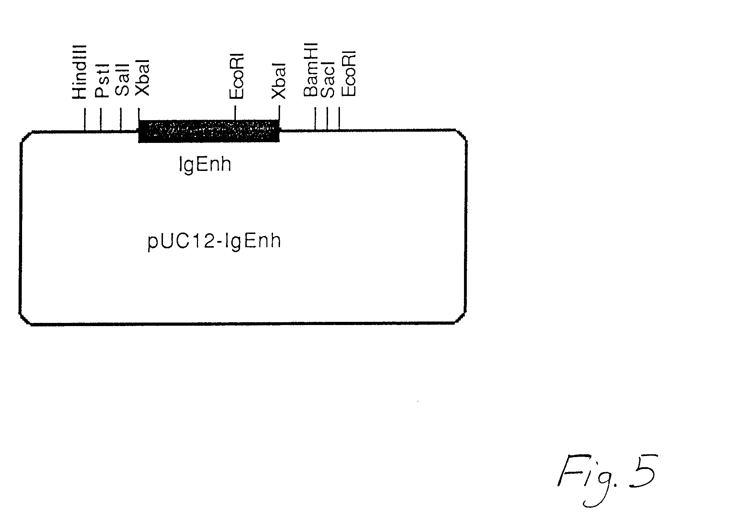

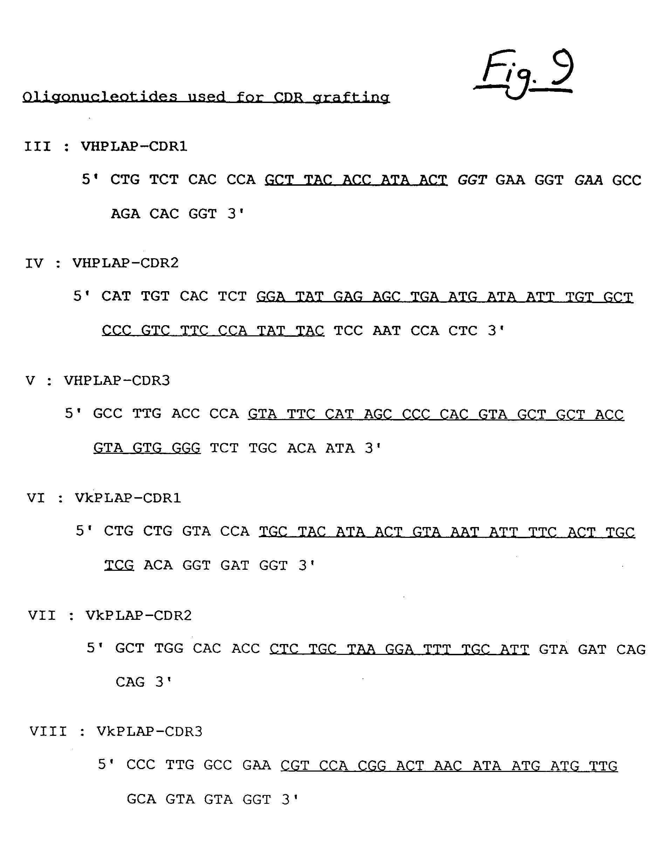

The CDRs in these constructs (Figures 3a and 4a) were replaced by site-directed mutagenesis with oligonucleotides encoding the anti-PLAP CDRs flanked by at least 12 nucleotides at each end encoding the corresponding human framework residues. These oligonucleotides are shown in Figure 9, in which the sequences corresponding to the CDRs are underlined.

In the present instance we found it useful also to conserve the amino acids Phe 27 and Thr 30 of the murine VHPLAP in the VH domain of the reshaped human anti-PLAP antibody. In oligonucleotide III, with 24 nucleotides flanking the 5' end of CDR 1, the murine Phe 27 and Thr 30 codons are shown in italics in Figure 9.

The mutagenesis was done as described in Riechmann et al (1988). The resulting variable regions were named Hu2VHPLAP and HuVkPLAP and are shown in Figure 10 and 11.

### c) Assembly of reshaped human antibody genes in expression vectors

The next stage involved the use of a murine heavy chain enhancer IgEnh, described in Neuberger et al (1983) where the enhancer is contained in a 1kb Xbal fragment of plasmid pSV-V»l. The 700bp Xbal/EcoRI subfragment of this 1kb Xbal fragment is sufficient to confer enhancer activity.

The reshaped human genes as prepared in section (b) above were excised from the M13 vectors as HindIII - BamHI fragments. The heavy chain variable region genes were cloned into a vector based on pSV2gpt (Mulligan et al, 1981), and the light chain variable region genes were cloned into a vector based on pSV2neo (Southern et al, 1981). Both contained the immunoglobulin heavy chain enhancer IgEnh. In the pSV2gpt based antibody expression vector (see Fig. 4b - 4c), the Xbal/EcoRI enhancer containing fragment was cloned in the unique EcoRI site of the pSV2gpt vector (after ligating EcoRI linkers to the filled in Xbal end of the fragment). The vector pSVgptMoVHLYS-MoIgG1 (Verhoeyen et al, 1988) was used as the source of a pSVgpt-based vector containing the IgEnh enhancer.

In the pSVneo based antibody expression vector (see Fig. 5a - 5b), the 1kb Xbal enhancer containing fragment was first cloned into pUC12 (Vieira et al, 1982), yielding the plasmid pUC12-IgEnh, see Figure 5. The enhancer can then be cut out as a 700bp EcoRI/HindIII fragment (either orientation of the enhancer will work), and cloned in the pSV2neo-derived vector (pSVneoMSN409 as shown in Figure 4a) obtained by removing the HindIII site in pSVneo. It is possible to use pSV2gpt as an alternative vector for light chain expression, as in practice there is no need for neo selection.

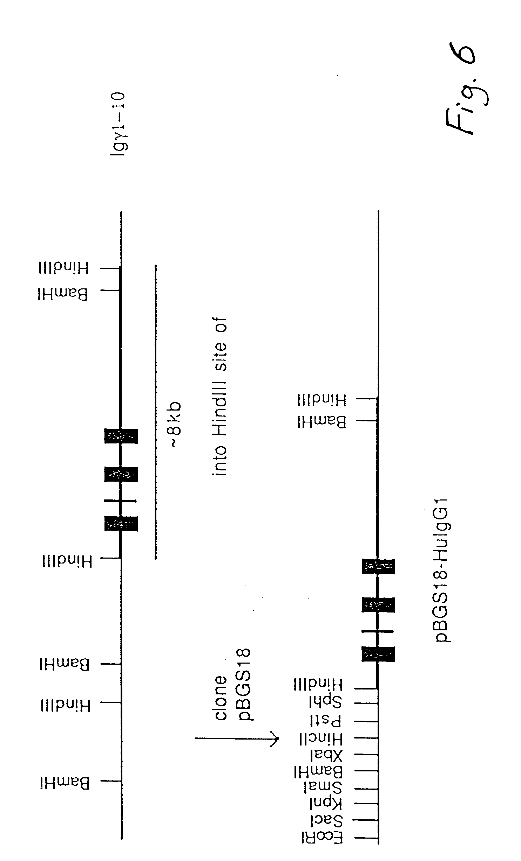

The Hu2VHPLAP gene was linked to a human gamma 1 constant region (Takahashi et al, 1982), cloned initially as a 8kb HindIII fragment into the HindIII site of pBGS18 (Spratt et al, 1986), and then in the pSV2gpt expression vector as a BamHI fragment (see Figures 3b and 6). It should be noted that in the Takahashi et al (1982) reference there is an error in Figure 1: the last (3') two sites are BamH1 followed by HindIII, and not the converse. This was confirmed by Flanagan et al (1982).

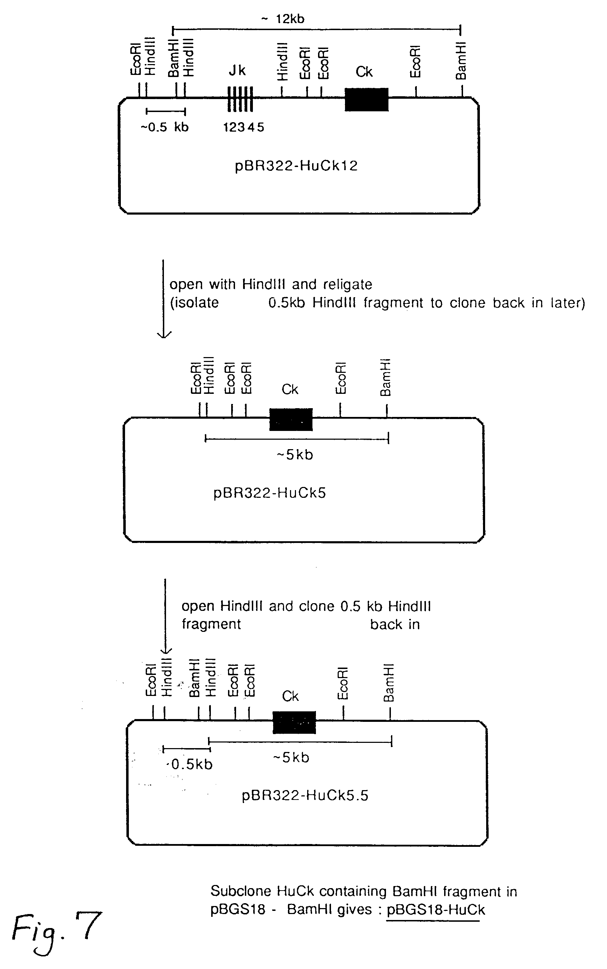

The HuVkPLAP gene was linked to a human C kappa constant region (Hieter et al, 1980) also cloned in as a BamHI fragment (see Figures 4b and 7). The source of the human Ck used in Figure 7 is given in Hieter et al (1980). The 12 kb BamH1 fragment from embryonic DNA (cloned in a gamma Ch28 vector system) was subcloned in the BamH1 site of plasmid pBR322.

### d) Expression in myeloma cells

Co-transfection of the expression plasmids pSVgptHu2VHPLAP-HuIgG1 and pSVneoHuVkPLAP-HuCk (Figures 3b and 4b) into NSO myeloma cells was done by electroporation (Potter et al, 1984), after linearisation with PvuI. Transfectomas were selected in mycophenolic acid containing medium to select for cells expressing the gpt gene product, and screened for antibody production and anti-PLAP activity by ELISA assays.

Positive clones were subcloned by limiting dilution and pure clones were assayed again for anti-PLAP activity, and the best producing clones were grown in serum-free medium for antibody production.

### e) Binding ability of the reshaped human antibodies

The practical application of the reshaped human antibody demands sufficient binding effectiveness. If the parent antibody has a very high effectiveness then some reduction during reshaping can be tolerated. The binding effectiveness will be dictated by many factors, one of which will be the antibody affinity for antigen, in this case placental alkaline phosphatase. A useful way of demonstrating binding ability of the reshaped antibody is to show that it has a similar antibody dilution curve when binding to antigen adsorbed on a plastic well surface. Such curves were generated as follows, using the parent murine anti-PLAP antibody and a reshaped human antibody prepared by the foregoing procedure.

Multiwell plates (Costar 6595, PETG) were coated with placental alkaline phosphatase (5 »g/ml in phosphate buffered saline pH 7.4, 37°C, 2 hours). The plates were rinsed in phosphate buffered saline before blocking with gelatin (0.02% in phosphate buffered saline) for one hour at room temperature, then washed four times with phosphate buffered saline with added Tween 20 (0.15%), and then used.

Antibody binding was performed in phosphate buffered saline with Tween 20 at room temperature for one hour, followed by four washes in buffer.

Visualisation of bound antibody was with horse radish peroxidase conjugated anti-globulins (anti-human IgG for the reshaped antibody and anti-mouse IgG for the parent molecule). The conjugate (Sigma) in buffer (1:1000) was incubated for one hour at room temperature, followed by four washes as above. Colour development (45 minutes) was with tetramethyl benzidene (0.01%) and hydrogen peroxide (1:200 or 100 vols) in citrate buffer pH6.5. The reaction was stopped with 2M hydrochloric acid.

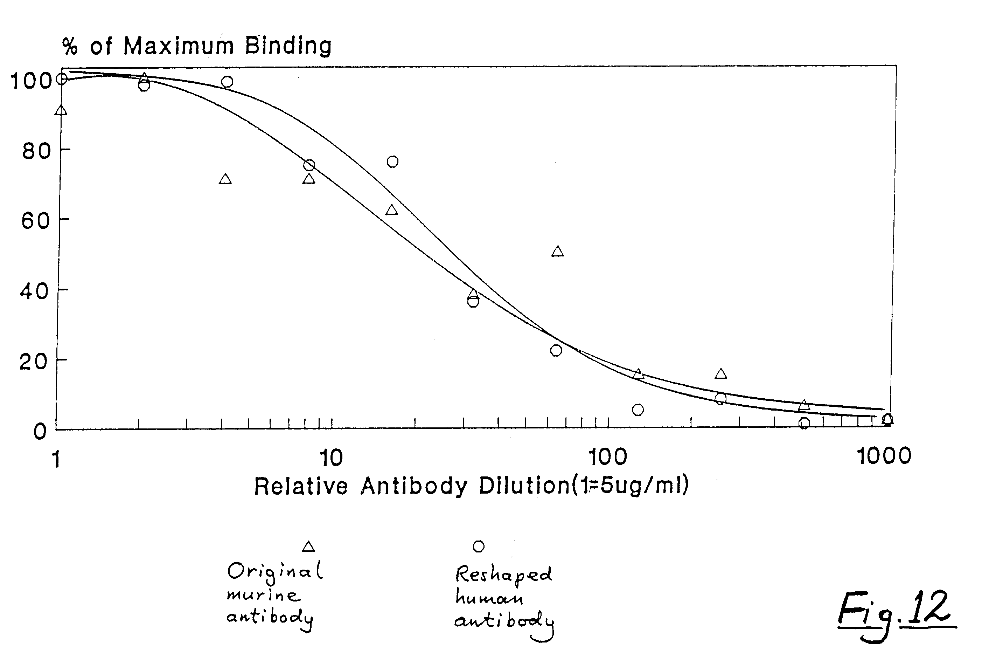

Controls showed insignificant colour due to non-specific binding of conjugate or due to binding of antibody to wells not containing placental alkaline phosphatase. The results, shown in Figure 12, are expressed as a percentage of the maximum colour (binding) seen. The two curves are similar, indicating a significant and useful level of binding effectiveness for the reshaped antibody of the invention.

### f) Deposited plasmids

E.coli strains containing plasmids used in the above procedure have been deposited, in accordance with the provisions of the Budapest Treaty, in the National Collection of Type Cultures on 19 April 1990 as follows:
- NCTC 12389:: K12, TG1 E.coli containing plasmid pSVgptHu2VHPLAP-HuIgG1
- NCTC 12390:: K12, TG1 E.coli containing plasmid pSVneoHuVkPLAP-HuCk

### References:

Deshpande et al (1990) - J. Nucl. Med., 31, p.473-479
Epp et al (1974) - Eur. J. Biochem. 45, p.513-524
Flanagan et al (1982) - Nature, 300, p.709-713
Gibson T (1984) - PhD thesis, LMB-MRC Cambridge
Gubler et al (1983) - Gene, 25, p.263-269
Hale et al (1988) - Lancet, 2, p.1394
Hieter et al (1980) - Cell, 22, p.197-207
Jones et al (1986) - Nature, 321, p.522-525
Kabat et al (1987) - in Sequences of Proteins of Immunological Interest, p.ix -US Dept of Health and Human Services
McCall et al (1990) - Bioconjugate Chemistry, 1, p.222-226
Meares et al (1990) - Br. J. Cancer, 62, Suppl. X, p.21-26
Moi et al (1988) - J. Am. Chem. Soc., 110, p.6266-6269
Mulligan et al (1981) - Proc. natn. Acad. Sci. U.S.A., 78 p.2072-2076
Neuberger et al (1983) - EMBO Journal, 2, p.1373-1378
Norrander et al (1983) - Gene, 26, p.101-106
Potter et al (1984) - PNAS, 81, p.7161-7163
Riechmann et al (1988) - Nature, 332, p.323-327
Sambrook et al (1989) - Molecular Cloning, 2nd Edition, Cold Spring Harbour Laboratory Press, New York
Sanger et al (1977) - PNAS USA, 74, p.5463-5467
Saul et al (1978) - J. biol. Chem. 253, p.585-597
Southern et al (1981) - J. molec. appl. Genetics, 1 p.327-345
Spratt et al (1986) - Gene, 41, p.337-342
Takahashi et al (1982) - Cell, 29, p.671-679
Travers et al (1984) - Int. J. Cancer, 33, p633
Verhoeyen et al (1988) - Science, 239, p.1534-2536
Vieira et al (1982) - Gene, 19, p.259-268
Winter (1987) - EP-A-239400

## Claims

1. A reshaped human antibody, or a reshaped human antibody fragment, having specificity for human placental alkaline phosphatase (PLAP) conferred by the presence of one or more of the amino acid sequences:
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr

2. A reshaped human antibody or reshaped human antibody fragment according to claim 1, having at least one heavy-chain variable region incorporating the following CDRs:
CDR1: Ser Tyr Gly Val Ser
CDR2: Val Ile Trp Glu Asp Gly Ser Thr Asn Tyr His Ser Ala Leu Ile Ser
CDR3: Pro His Tyr Gly Ser Ser Tyr Val Gly Ala Met Glu Tyr

3. A reshaped human antibody or reshaped human antibody fragment according to claim 1, having at least one light-chain variable region incorporating the following CDRs:
CDR1: Arg Ala Ser Glu Asn Ile Tyr Ser Tyr Val Ala
CDR2: Asn Ala Lys Ser Leu Ala Glu
CDR3: Gln His His Tyr Val Ser Pro Trp Thr

4. A reshaped human antibody or reshaped human antibody fragment and having at least one heavy-chain variable region according to claim 2 and at least one light-chain variable region according to claim 3.

5. A reshaped human antibody or reshaped human antibody fragment incorporating at least one heavy-chain variable region comprising the entire amino acid sequence depicted in Figure 10 of the accompanying drawings.

6. A reshaped human antibody or reshaped human antibody fragment incorporating at least one light-chain variable region comprising the entire amino acid sequence depicted in Figure 11 of the accompanying drawings.

7. A reshaped human antibody or a reshaped human antibody fragment, having specificity equivalent to that of the gamma-1, kappa anti-PLAP monoclonal antibody secreted by murine hybridoma cell line H17E2.

8. A stable host cell line producing a reshaped human antibody or a reshaped human antibody fragment according to any one of claims 1 to 7, resulting from incorporation in the cell line of a foreign gene encoding the reshaped human antibody or reshaped human antibody fragment.

9. A stable host cell line according to claim 8, wherein the foreign gene includes one or more of the nucleotide sequences:
i) AGT TAT GGT GTA AGC
ii) GTA ATA TGG GAA GAC GGG AGC ACA AAT TAT CAT TCA GCT CTC ATA TCC
iii) CCC CAC TAC GGT AGC AGC TAC GTG GGG GCT ATG GAA TAC
iv) CGA GCA AGT GAA AAT ATT TAC AGT TAT GTA GCA
v) AAT GCA AAA TCC TTA GCA GAG
vi) CAA CAT CAT TAT GTT AGT CCG TGG ACG

10. A stable host celll line according to claim 8, wherein the foreign gene includes the entire nucleotide sequence depicted in Figure 10 of the accompanying drawings.

11. A stable host cell line according to claim 8, wherein the foreign gene includes the entire nucleotide sequence depicted in Figure 11 of the accompanying drawings.

12. A stable host cell line according to claim 8, wherein the foreign gene encodes:
a) at least one of the amino acid sequences:
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr
and b) a protein framework that enables the encoded amino acid sequence when expressed to function as a CDR having specificity for human placental alkaline phosphatase.

13. A stable host cell line according to claim 8, wherein the foreign gene encodes the entire amino acid sequence depicted in Figure 10 of the accompanying drawings.

14. A stable host cell line according to claim 8, wherein the foreign gene encodes the entire amino acid sequence depicted in Figure 11 of the accompanying drawings.

15. Plasmid pSVgptHu2VHPLAP-HuIgG1, as contained in E.coli NCTC 12389.

16. Plasmid pSVneoHuVkPLAP-HuCk, as contained in E.coli NCTC 12390.

17. Use of a plasmid according to claim 15 or claim 16 in the production of a reshaped human antibody or reshaped human antibody fragment.

18. E.coli NCTC 12389.

19. E.coli NCTC 12390.

20. A DNA sequence encoding a reshaped human antibody heavy-chain variable region having specificity for human placental alkaline phosphatase, as contained in E.coli NCTC 12389.

21. A DNA sequence encoding a reshaped human antibody light-chain variable region having specificity for human placental alkaline phosphatase, as contained in E.coli NCTC 12390.

22. A reshaped human antibody heavy-chain variable region having specificity for human placental alkaline phosphatase, producible by means of the expression vector contained in E.coli NCTC 12389.

23. A reshaped human antibody light-chain variable region having specificity for human alkaline phosphatase, producible by means of the expression vector contained in E.coli NCTC 12390.

24. A reshaped human antibody or reshaped human antibody fragment, comprising at least one variable region according to claim 22 or claim 23.

25. A reshaped human antibody or reshaped human antibody fragment, according to any one of claims 1 to 7 or claim 24, linked to or incorporating an agent capable of retarding or terminating the growth of cancerous cells, or linked to an agent capable of being detected while inside the human body.

26. An injectable composition comprising a reshaped human antibody or reshaped human antibody fragment, according to claim 25, in a pharmaceutically acceptable carrier.

27. Use of a reshaped human antibody or reshaped human antibody fragment, according to any one of claims 1 to 7 or claim 24, for the manufacture of a medicament for therapeutic application in the relief of cancer in humans, or for the manufacture of a diagnostic composition for in-vivo diagnostic application in humans.

## Patentansprüche

1. Umgebauter bzw. reshaped humaner Antikörper oder umgebautes bzw. reshaped humanes Antikörperfragment mit Spezifität auf humane plazentale alkalische Phosphatase (PLAP), die durch die Anwesenheit einer oder mehrerer der folgenden Aminosäuresequenzen verliehen wird:
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr

2. Reshaped humaner Antikörper oder reshaped humanes Antikörperfragment nach Anspruch 1, worin mindestens eine variable Region einer schweren Kette die folgenden CDRs enthält:
CDR1: Ser Tyr Gly Val Ser
CDR2: Val Ile Trp Glu Asp Gly Ser Thr Asn Tyr His Ser Ala Leu Ile Ser
CDR3: Pro His Tyr Gly Ser Ser Tyr Val Gly Ala Met Glu Tyr

3. Reshaped humaner Antikörper oder reshaped humanes Antikörperfragment nach Anspruch 1, worin mindestens eine variable Region einer leichten Kette die folgenden CDRs enthält:
CDR1: Arg Ala Ser Glu Asn Ile Tyr Ser Tyr Val Ala
CDR2: Asn Ala Lys Ser Leu Ala Glu
CDR3: Gln His His Tyr Val Ser Pro Trp Thr

4. Reshaped humaner Antikörper oder reshaped humanes Antikörperfragment, worin mindestens eine variable Region einer schweren Kette nach Anspruch 2 und mindestens eine variable Region einer leichten Kette nach Anspruch 3 enthalten ist.

5. Reshaped humaner Antikörper oder reshaped humanes Antikörperfragment, worin mindestens eine variable Region einer schweren Kette die vollständige in Fig. 10 der anliegenden Abbildungen dargestellte Aminosäuresequenz umfaßt.

6. Reshaped humaner Antikörper oder reshaped humanes Antikörperfragment, worin mindestens eine variable Region einer leichten Kette die vollständige in Fig. 11 der anliegenden Abbildungen dargestellte Aminosäuresequenz umfaßt.

7. Reshaped humaner Antikörper oder reshaped humanes Antikörperfragment mit Spezifität, die derjenigen des monoklonalen Gamma-1, Kappa-anti-PLAP-Antikörpers entspricht, der von der Maushybridomzellinie H17E2 sekretiert wird.

8. Stabile, einen reshaped humanen Antikörper oder ein reshaped humanes Antikörperfragment nach einem der Ansprüche 1 bis 7 produzierende Wirtszellinie, die aus der Einführung eines fremden Gens, welches für den reshaped humanen Antikörper oder das reshaped humane Antikörperfragment kodiert, in die Zellinie hervorgegangen ist.

9. Stabile Wirtszellinie nach Anspruch 8, worin das fremde Gen eine oder mehrere der Nukleotidsequenzen einschließt:
i) AGT TAT GGT GTA AGC
ii) GTA ATA TGG GAA GAC GGG AGC ACA AAT TAT CAT TCA GCT CTC ATA TCC
iii) CCC CAC TAC GGT AGC AGC TAC GTG GGG GCT ATG GAA TAC
iv) CGA GCA AGT GAA AAT ATT TAC AGT TAT GTA GCA
v) AAT GCA AAA TCC TTA GCA GAG
vi) CAA CAT CAT TAT GTT AGT CCG TGG ACG

10. Stabile Wirtszellinie nach Anspruch 8, worin das fremde Gen die vollständige in Fig. 10 der anliegenden Abbildungen dargestellte Nukleotidsequenz einschließt.

11. Stabile Wirtszellinie nach Anspruch 8, worin das fremde Gen die vollständige in Fig. 11 der anliegenden Abbildungen dargestellte Nukleotidsequenz einschließt.

12. Stabile Wirtszellinie nach Anspruch 8, worin das fremde Gen kodiert für:
a) mindestens eine der Aminosäuresequenzen:
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr
und b) einen Protein-Rahmenbezirk, der die kodierte Aminosäuresequenz in die Lage versetzt, nach der Expression als CDR mit Spezifität auf humane plazentale alkalische Phosphatase zu wirken.

13. Stabile Wirtszellinie nach Anspruch 8, worin das fremde Gen für die gesamte in Fig. 10 der anliegenden Abbildungen dargestellte Aminosäuresequenz kodiert.

14. Stabile Wirtszellinie nach Anspruch 8, worin das fremde Gen für die gesamte in Fig. 11 der anliegenden Abbildungen dargestellte Aminosäuresequenz kodiert.

15. Plasmid pSVgptHu2VHPLAP-HuIgG1, wie in E.coli NCTC 12389 enthalten.

16. Plasmid pSVneoHuVkPLAP-HuCk, wie in E.coli NCTC 12390 enthalten.

17. Verwendung eines Plasmids nach Anspruch 15 oder 16 bei der Herstellung eines reshaped humanen Antikörpers oder reshaped humanen Antikörperfragments.

18. E.coli NCTC 12389.

19. E.coli NCTC 12390.

20. DNA-Sequenz, die für die variable Region einer schweren Kette eines reshaped humanen Antikörpers mit Spezifität auf humane plazentale alkalische Phosphatase kodiert, wie in E.coli NCTC 12389 enthalten.

21. DNA-Sequenz, die für die variable Region einer leichten Kette eines reshaped humanen Antikörpers mit Spezifität auf humane plazentale alkalische Phosphatase kodiert, wie in E.coli NCTC 12390 enthalten.

22. Variable Region einer schweren Kette eines reshaped humanen Antikörpers mit Spezifität auf humane plazentale alkalische Phosphatase, herstellbar mittels des in E.coli NCTC 12389 enthaltenen Expressionsvektors.

23. Variable Region einer leichten Kette eines reshaped humanen Antikörpers mit Spezifität auf humane plazentale alkalische Phosphatase, herstellbar mittels des in E.coli NCTC 12390 enthaltenen Expressionsvektors.

24. Reshaped humaner Antikörper oder reshaped humanes Antikörperfragment, der/das mindestens eine variable Region nach Anspruch 22 oder 23 umfaßt.

25. Reshaped humaner Antikörper oder reshaped humanes Antikörperfragment nach einem der Ansprüche 1 bis 7 oder 24, der/das an ein Reagenz gebunden ist oder dieses enthält, welches fähig ist, das Wachstum von Krebszellen zu verlangsamen oder zu beenden, oder an ein Reagenz gebunden ist, welches im Innern des menschlichen Körpers nachgewiesen werden kann.

26. Injizierbare Zusammensetzung, die einen reshaped humanen Antikörper oder ein reshaped humanes Antikörperfragment nach Anspruch 25 in einem pharmazeutischen zulässigen Trägermaterial umfaßt.

27. Verwendung eines reshaped humanen Antikörpers oder reshaped humanen Antikörperfragments nach einem der Ansprüche 1 bis 7 oder 24 zur Herstellung eines Arzneimittels zur therapeutischen Anwendung bei der Linderung von Krebs beim Menschen oder zur Herstellung einer diagnostischen Zusammensetzung zur in vivo-diagnostischen Anwendung bei Menschen.

## Revendications

1. Anticorps humain refaçonné, ou fragment d'anticorps humain refaçonné, ayant une spécificité pour la phosphatase alcaline placentaire humaine (PLAP) conférée par la présence d'une ou plusieurs des séquences d'acides aminés suivantes :
i) Ser Tyr Gly Val Ser
ii) Val Ile Trp Glu Asp Gly Ser Thr Asn Tyr His Ser Ala Leu Ile Ser
iii) Pro His Tyr Gly Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr

2. Anticorps humain refaçonné ou fragment d'anticorps humain refaçonné selon la revendication 1, ayant au moins une région variable à chaîne lourde incorporant les régions déterminant la complémentarité (CDR) suivante :
CDR1 : Ser Tyr Gly Val Ser
CDR2 : Val Ile Trp Glu Asp Gly Ser Thr Asn Tyr His Ser Ala Leu Ile Ser
CDR3 : Pro His Tyr Gly Ser Ser Tyr Val Gly Ala Met Glu Tyr

3. Anticorps humain refaçonné ou fragment d'anticorps humain refaçonné selon la revendication 1, ayant au moins une région variable à chaîne légère incorporant les CDR suivantes :
CDR1 : Arg Ala Ser Glu Asn Ile Tyr Ser Tyr Val Ala
CDR2 : Asn Ala Lys Ser Leu Ala Glu
CDR3 : Gln His His Tyr Val Ser Pro Trp Thr

4. Anticorps humain refaçonné ou fragment d'anticorps humain refaçonné et ayant au moins une région variable à chaîne lourde selon la revendication 2 et au moins une région variable à chaîne légère selon la revendication 3.

5. Anticorps humain refaçonné ou fragment d'anticorps humain refaçonné incorporant au moins une région variable à chaîne lourde comprenant la séquence entière d'acides aminés représentée dans la figure 10 des dessins joints.

6. Anticorps humain refaçonné ou fragment d'anticorps humain refaçonné incorporant au moins une région variable à chaîne légère comprenant la séquence entière d'acides aminés représentée dans la figure 11 des dessins joints.

7. Anticorps humain refaçonné ou fragment d'anticorps humain refaçonné ayant une spécificité équivalant à celle de l'anticorps monoclonal anti-PLAP gamma-1, kappa sécrété par la lignée cellulaire d'hybridome murin H17E2.

8. Lignée cellulaire hôte stable produisant un anticorps humain refaçonné ou un fragment d'anticorps humain refaçonné selon l'une quelconque des revendications 1 à 7, résultant de l'incorporation dans la lignée cellulaire d'un gène étranger codant pour l'anticorps humain refaçonné ou le fragment d'anticorps humain refaçonné.

9. Lignée cellulaire hôte stable selon la revendication 8, dans laquelle le gène étranger inclut une ou plusieurs des séquences nucléotidiques suivantes :
i) AGT TAT GGT GTA AGC
ii) GTA ATA TGG GAA GAC GGG AGC ACA AAT TAT CAT TCA GCT CTC ATA TCC
iii) CCC CAC TAC GGT AGC AGC TAC GTG GGG GCT ATG GAA TAC
iv) CGA GCA AGT GAA AAT ATT ATT TAC AGT TAT GTA GCA
v) AAT GCA AAA TCC TTA GCA GAG
vi) CAA CAT CAT TAT GTT AGT CCG TGG ACG

10. Lignée cellulaire hôte selon la revendication 8, dans laquelle le gène étranger inclut la séquence nucléotidique entière représentée par la figure 10 des dessins joints.

11. Lignée cellulaire hôte stable selon la revendication 8, dans laquelle le gène étranger inclut la séquence nucléotidique entière représentée dans la figure 11 des dessins joints.

12. Lignée cellulaire hôte stable selon la revendication 8, dans laquelle le gène étranger code pour :
a) au moins l'une des séquences d'acides aminés suivantes :
i) Ser Tyr Gly Val Ser
ii) Val Ile Trp Glu Asp Gly Ser Thr Asn Tyr His Ser Ala Leu Ile Ser
iii) Pro His Tyr Gly Ser Ser Tyr Val Gly Ala Met Glu Tyr
iv) Arg Ala Ser Glu Asn Ile Tyr Ser Tyr Val Ala
v) Asn Ala Lys Ser Leu Ala Glu
vi) Gln His His Tyr Val Ser Pro Trp Thr
et b) une ossature de protéine qui permet à la séquence d'acides aminés encodée lorsqu'elle est exprimée de fonctionner comme CDR ayant une spécificité pour la phosphatase alcaline placentaire humaine.

13. Lignée cellulaire hôte stable selon la revendication 8, dans laquelle le gène étranger code pour la séquence entière d'acides aminés représentée dans la figure 10 des dessins joints.

14. Lignée cellulaire hôte stable selon la revendication 8, dans laquelle le gène étranger code pour la séquence entière d'acides aminés représentée dans la figure 11 des dessins joints.

15. Plasmide pSVgptHu2VHPLAP-HuIgG1, tel que contenu dans E. coli NCTC 12389.

16. Plasmide pSVneoHuVkPLAP-HuCk, tel que contenu dans E. coli. NCTC 12390.

17. Application d'un plasmide selon la revendication 15 ou la revendication 16 dans la préparation d'un anticorps humain refaçonné ou d'un fragment d'un anticorps humain refaçonné.

18. E. coli NCTC 12389.

19. E. coli NCTC 12390.

20. Séquence d'ADN codant pour une région variable à chaîne lourde d'anticorps humain refaçonné ayant une spécificité pour la phosphatase alcaline placentaire humaine, telle que contenue dans E. coli NCTC 12389.

21. Séquence d'ADN codant pour une région variable à chaîne lourde d'anticorps humain refaçonné ayant une spécificité pour la phosphatase alcaline placentaire humaine, telle que contenue dans E. coli NCTC 12390.

22. Région variable à chaîne lourde d'anticorps humain refaçonnée ayant une spécificité pour la phosphatase alcaline placentaire humaine, pouvant être produite au moyen du vecteur d'expression contenu dans E. coli NCTC 12389.

23. Région variable à chaîne légère d'anticorps humain refaçonnée ayant une spécificité pour la phosphatase alcaline placentaire humaine, pouvant être produite au moyen du vecteur d'expression contenu dans E. coli NCTC 12390.

24. Anticorps humain refaçonné ou fragment d'anticorps humain refaçonné comprenant au moins une région variable selon la revendication 22 ou la revendication 23.

25. Anticorps humain refaçonné ou fragment d'anticorps refaçonné selon l'une quelconque des revendications 1 à 7 ou la revendication 24, lié à ou incorporant un agent capable de retarder ou de mettre fin à la croissance des cellules cancéreuses, ou lié à un agent pouvant être détecté lorsqu'il est à l'intérieur du corps humain.

26. Composition injectable comprenant un anticorps humain refaçonné ou un fragment d'anticorps humain refaçonné selon la revendication 25 dans un support pharmaceutiquement acceptable.

27. Application d'un anticorps humain refaçonné ou d'un fragment d'anticorps humain refaçonné selon l'une quelconque des revendications 1 à 7 ou la revendication 29, à la préparation d'un médicament pour application thérapeutique afin de soulager le cancer chez l'homme, ou pour préparer une composition de diagnostic pour une application de diagnostic in-vivo chez l'homme.
